# EUROPEAN PATENT SPECIFICATION

(11) **EP 2 508 509 B1**
(45) Date of publication and mention of the grant of the patent: **17.08.2016**
(21) Application number: 10834691.7
(22) Date of filing: 01.12.2010
(51) Int. Cl.: C07C 253/30, C07C 255/31, C07B 35/08, C07B 61/00

(54) **PROCESS FOR PREPARATION OF (Z)-CYANOALKENYLCYCLOPROPANECARBOXYLIC ACID COMPOUNDS**
VERFAHREN ZUR HERSTELLUNG VON (Z)-CYAN-ALKENYL-CYCLOPROPAN-CARBONSÄURE-VERBINDUNGEN
PROCÉDÉ DE PRÉPARATION DE COMPOSÉS ACIDES (Z)-CYANOALCÉNYLCYCLOPROPANECARBOXYLIQUES

(30) Priority: 02.12.2009 JP 2009274313; 02.12.2009 JP 2009274314
(43) Date of publication of application: 10.10.2012
(73) Proprietor: Sumitomo Chemical Company, Limited, Tokyo 104-8260 (JP)
(72) Inventor: SOMYO, Toshio, Ibaraki-shi Osaka 567-0841 (JP); UEKAWA, Toru, Toyonaka-shi Osaka 560-0021 (JP); MORI, Tatsuya, Toyonaka-shi Osaka 561-0802 (JP)
(74) Representative: Vossius & Partner Patentanwälte Rechtsanwälte mbB
(86) International application number: PCT/JP2010/071951
(87) International publication number: WO 2011/068244

(56) References cited:
- EP-A1- 2 537 828
- JP-A- 2004 002 363
- JP-A- 2008 239 597
- JP-A- 2009 185 019
- US-A1- 2003 195 119
- UEDA KENZO ET AL.: 'Studies on Chrysanthemic Acid - Part XX. Synthesis of Four Geometrical Isomers of (+-)-Pyrethric Acid' AGRICULTURAL AND BIOLOGICAL CHEMISTRY vol. 34, no. 7, 1970, pages 1119 - 1125, XP055094130
- TORTAJADA JEANINE ET AL.: 'Preparation d'olefines trisubstituees bifonctionnelles par voie photochimique' TETRAHEDRON vol. 40, no. 3, 1984, pages 613 - 619, XP026658894
- ARMESTO DIEGO ET AL.: 'Photochemistry of BETA, y-unsaturated oxime acetates. Aza-dl-TT-methane reactivity offunctionalised all-aliphatic systems. A photochemical approach to pyrethrin-likecyclopropane derivatives-LIKE CYCLOPROPANE DERIVATIVES' TETRAHEDRON vol. 46, no. 17, 1990, pages 6185 - 6192, XP055094895

## Description

### TECHNICAL FIELD

The present invention relates to a method for producing a (Z)-cyanoalkenylcyclopropanecarboxylic acid compound.

### BACKGROUND ART

It is known that a (Z)-cyanoalkenyl-cyclopropanecarboxylic acid compound represented by formula (2) (hereinafter sometimes referred to as compound (2)): 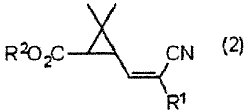 wherein R¹ represents an alkyl group which may have a substituent, or a halogen atom, and R² represents a hydrogen atom, an alkyl group which may have a substituent, or a benzyl group which may have a substituent, is useful as an agent for controlling noxious organisms such as pests (see, for example, JP-A-2004-2363 and JP-A-2008-239597).

Agr. Biol. Chem., 34, 1119 (1970) describes, as a method for producing the compound (2), for example, a method in which a (Z)-3-(2-cyano-1-propenyl)-2,2-dimethylcyclopropanecarboxylic acid ester is obtained by reacting a cyclopropane aldehyde compound with a phosphonate compound (carrying out a so-called Honer-Wadsworth-Emmons reaction), and a method in which (Z)-3-(2-cyano-1-propenyl)-2,2-dimethylcyclopropanecarboxylic acid is obtained by subjecting the obtained (Z)-3-(2-cyano-1-propenyl)-2,2-dimethylcyclopropanecarboxylic acid ester to ester hydrolysis.

In the method described in Agr. Biol. Chem., 34, 1119 (1970), a geometric isomer of the compound (2), i.e., an (E)-cyanoalkenylcyclopropanecarboxylic acid compound represented by formula (1) (hereinafter sometimes referred to as compound (1)): 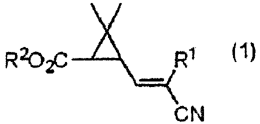 wherein R¹ and R² has the same meanings as defined above, may sometimes be produced in a higher yield as compared with the compound (2). However, there has never been known a method in which the compound (2) is obtained from the compound (1).

### DISCLOSURE OF THE INVENTION

The present inventors intensively studied and thus the present invention has been completed. Namely, the present invention includes the following.
[1] A method for producing a (Z)-cyanoalkenyl-cyclopropanecarboxylic acid compound, which method comprises the step of isomerizing an (E)-cyanoalkenyl-cyclopropanecarboxylic acid compound represented by formula (1) (hereinafter sometimes referred to as compound (1)): 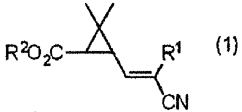 wherein R¹ represents an alkyl group which may have a substituent, or a halogen atom, and R² represents a hydrogen atom, an alkyl group which may have a substituent, or a benzyl group which may have a substituent, into a (Z)-cyanoalkenyl-cyclopropanecarboxylic acid compound represented by formula (2) (hereinafter sometimes referred to as compound (2)): 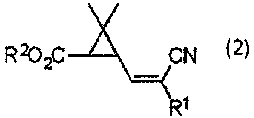 wherein R¹ and R² have the same meanings as defined above, in the presence of at least one isomerizing catalyst selected from the group consisting of bromine, hydrogen bromide, brominated carboxylic acids, brominated phosphorus compounds, N-brominated imide compounds, N-brominated amide compounds, brominated alkylsilane compounds, thionyl bromide, brominated boron compounds, brominated aluminum compounds, thiol compounds, disulfide compounds, thiocarboxylic acid compounds, nitric acid and nitrate salts.
[2] The method according to [1], wherein the above described step is the step of isomerizing the (E)-cyanoalkenyl-cyclopropanecarboxylic acid compound into the (Z)-cyanoalkenyl-cyclopropanecarboxylic acid compound further in the presence of a radical initiator.
[3] The method according to [2], wherein the radical initiator is at least one radical initiator selected from the group consisting of inorganic peroxides, ketone peroxide compounds, hydroperoxide compounds, diacyl peroxide compounds, peracid ester compounds, peracid compounds and azo compounds.
[4] The method according to [1] or [2], wherein R² represents a hydrogen atom.
[5] The method according to [1] or [2], wherein R² represents an alkyl group which may have a substituent, or a benzyl group which may have a substituent.
[6] A method for isomerizing an (E)-cyanoalkenyl-cyclopropanecarboxylic acid compound, which comprises isomerizing an (E)-cyanoalkenyl-cyclopropanecarboxylic acid compound represented by formula (1): 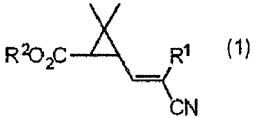 wherein R¹ represents an alkyl group which may have a substituent, or a halogen atom, and R² represents a hydrogen atom, an alkyl group which may have a substituent, or a benzyl group which may have a substituent, into a (Z)-cyanoalkenyl-cyclopropanecarboxylic acid compound represented by formula (2): 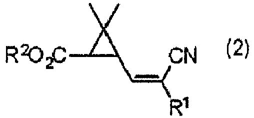 wherein R¹ and R² have the same meanings as defined above, in the presence of at least one isomerizing catalyst selected from the group consisting of bromine, hydrogen bromide, brominated carboxylic acids, brominated phosphorus compounds, N-brominated imide compounds, N-brominated amide compounds, brominated alkylsilane compounds, thionyl bromide, brominated boron compounds, brominated aluminum compounds, thiol compounds, disulfide compounds, thiocarboxylic acid compounds, nitric acid and nitrate salts.

### MODE FOR CARRYING OUT THE INVENTION

The present invention will be described in detail below.

The production method of the present invention comprises the step of isomerizing the compound (1) into the compound (2) in the presence of at least one isomerizing catalyst selected from the group consisting of bromine, hydrogen bromide, brominated carboxylic acids, brominated phosphorus compounds, N-brominated imide compounds, N-brominated amide compounds, brominated alkylsilane compounds, thionyl bromide, brominated boron compounds, brominated aluminum compounds, thiol compounds, disulfide compounds, thiocarboxylic acid compounds, nitric acid and nitrate salts. Also, the isomerization method of the present invention includes isomerizing the compound (1) into the compound (2) in the presence of the above isomerizing catalyst. Hereinafter, the step of isomerizing the compound (1) into the compound (2) and the isomerization of the compound (1) into the compound (2) are sometimes referred to as "isomerization step".

First, the compound (1) used in the isomerization step will be described. With respect to a geometric isomer based on the cyclopropane ring, the compound (1) may be either a cis-isomer or a trans-isomer, or may be a mixture of a cis-isomer and a trans-isomer in any ratio. In the compound (1), two carbon atoms of the carbon atoms constituting the cyclopropane ring are asymmetric carbons, and the compound (1) may be a compound which exhibits optical activity or a compound which does not exhibit optical activity.

The compound (1) is represented by formula (1).

In formula (1), R¹ represents an alkyl group which may have a substituent, or a halogen atom.

Examples of the halogen atom represented by R¹ include a fluorine atom, a chlorine atom, a bromine atom and an iodine atom, and preferably a chlorine atom and a bromine atom.

In the alkyl group which may have a substituent and is represented by R¹, the alkyl group means a linear alkyl group having about 1 to about 10 carbon atoms, a branched alkyl group having about 3 to about 10 carbon atoms, or a cycloalkyl group having about 3 to about 10 carbon atoms.

Examples of the linear alkyl group having about 1 to about 10 carbon atoms include a methyl group, an ethyl group, a n-propyl group and a n-butyl group; examples of the branched alkyl group having about 3 to about 10 carbon atoms include an isopropyl group, an isobutyl group, a t-butyl group, an amyl group and a 2-ethylhexyl group; and examples of the cycloalkyl group having about 3 to about 10 carbon atoms include a cyclopropyl group, a cyclopentyl group and a cyclohexyl group.

Examples of the substituent which may be contained in the alkyl group include a halogen atom and an alkoxy group having about 1 to about 4 carbon atoms. Examples of the alkyl group having a substituent include a fluoromethyl group, a chloromethyl group, a trifluoromethyl group and a trichloromethyl group.

Preferably, examples of R¹ include a linear alkyl group having 1 to 4 carbon atoms and a halogen atom, more preferably a linear alkyl group having 1 to 4 carbon atoms, a chlorine atom and a bromine atom, and still more preferably a methyl group and a chlorine atom.

In formula (1), R² represents a hydrogen atom, an alkyl group which may have a substituent, or a benzyl group which may have a substituent.

In the alkyl group which may have a substituent and is represented by R², the alkyl group may be an alkyl group that in the alkyle group which may have a substituent and is represented by R¹.

In R², examples of the substituent which may be contained in the alkyl group include:
halogen atoms, such as a fluorine atom, a chlorine atom and a bromine atom;
alkenyl groups having about 3 to about 4 carbon atoms, such as an allyl group;
alkynyl groups having about 3 to about 4 carbon atoms, such as a propargyl group;
alkoxy groups having about 1 to about 4 carbon atoms, such as a methoxy group and an ethoxy group; and
alkylthio groups having about 1 to about 4 carbon atoms, such as a methylthio group.

In R², examples of the substituent which may be contained in the benzyl group include:
halogen atoms, such as a fluorine atom, a chlorine atom and a bromine atom;
alkenyl groups having about 3 to about 4 carbon atoms, such as an allyl group;
alkynyl groups having about 3 to about 4 carbon atoms, such as a propargyl group;
alkoxy groups having about 1 to about 4 carbon atoms, such as a methoxy group and an ethoxy group;
alkylthio groups having about 1 to about 4 carbon atoms, such as a methylthio group;
linear alkyl groups having about 1 to about 10 carbon atoms, such as a methyl group, an ethyl group and an n-propyl group;
branched alkyl groups having about 3 to about 10 carbon atoms, such as an isopropyl group, an isobutyl group, a t-butyl group, an amyl group and a 2-ethylhexyl group;
cycloalkyl groups having about 3 to about 10 carbon atoms, such as a cyclopropyl group, a cyclopentyl group and a cyclohexyl group;
alkoxymethyl groups having about 1 to about 4 carbon atoms, such as a methoxymethyl group and an ethoxymethyl group; and
alkylthiomethyl groups having about 1 to about 4 carbon atoms, such as a methylthiomethyl group.

Preferably, examples of R² include hydrogen, a linear alkyl group having 1 to 4 carbon atoms or a benzyl group which may have a substituent, more preferably hydrogen, a linear alkyl group having 1 to 4 carbon atoms, and a benzyl group which may have a halogen atom, an alkenyl group having about 3 to about 4 carbon atoms, an alkynyl group having about 3 to about 4 carbon atoms, an alkoxymethyl group having about 1 to about 4 carbon atoms or an alkylthiomethyl group having about 1 to about 4 carbon atoms, and still more preferably hydrogen, a methyl group, an ethyl group, a benzyl group, a 4-methyl-2,3,5,6-tetrafluorobenzyl group, a 4-methoxymethyl-2,3,5,6-tetrafluorobenzyl group, a 4-allyl-2,3,5,6-tetrafluorobenzyl group, a 4-propargyl-2,3,5,6-tetrafluorobenzyl group and a 4-methylthiomethyl-2,3,5,6-tetrafluorobenzyl group.

Examples of the compound (1) include the compounds indicated the numbers (1-1) to (1-176) shown in Table 1, Table 2 and Table 3.

**Table 1**

| | | | | | | | | |
|---|---|---|---|---|---|---|---|---|
| 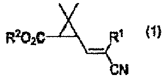 | | | | | | | | |

| Number | R¹ | R² | Number | R¹ | R² | Number | R¹ | R² |
|---|---|---|---|---|---|---|---|---|
| (1-1) | -CH₃ | -CH₃ | (1-21) | -CH₃ | -CH₂CH₃ | (1-41) | -CH₃ | 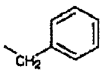 |
| (1-2) | -CH₂CH₃ | -CH₃ | (1-22) | -CH₂CH₃ | -CH₂CH₃ | (1-42) | -CH₂CH₃ | 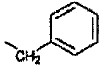 |
| (1-3) | -CH₂CH₂CH₃ | -CH₃ | (1-23) | -CH₂CH₂CH₃ | -CH₂CH₃ | (1-43) | -CH₁CH₂CH₃ | 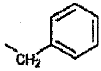 |
| (1-4) | 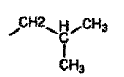 | -CH₃ | (1-24) | 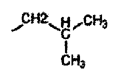 | -CH₂CH₃ | (1-44) | 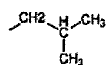 | 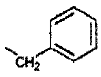 |
| (1-5) | 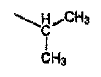 | -CH₃ | (1-25) | 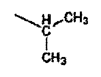 | -CH₂CH₃ | (1-45) | 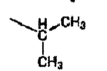 | 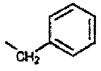 |
| (1-6) | 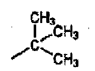 | -CH₃ | (1-26) | 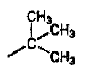 | -CH₂CH₃ | (1-46) | 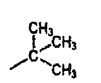 | 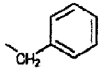 |
| (1-7) | 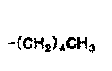 | -CH₃ | (1-27) | -(CH₂)₄CH₃ | -CH₂CH₃ | (1-47) | -(CH₂)₄CH₃ | 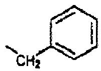 |
| (1-8) | 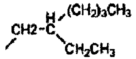 | -CH₃ | (1-28) | 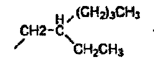 | -CH₂CH₃ | (1-48) | 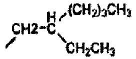 | 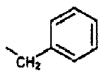 |
| (1-9) | 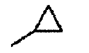 | -CH₃ | (1-29) | 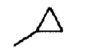 | -CH₂CH₃ | (1-49) | 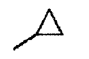 | 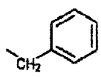 |
| (1-10) | 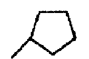 | -CH₃ | (1-30) | 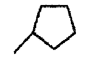 | -CH₂CH₃ | (1-50) | 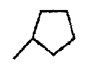 | 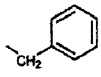 |
| (1-11) | 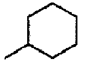 | -CH₃ | (1-31) | 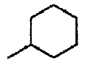 | -CH₂CH₃ | (1-51) | 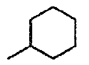 | 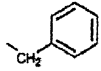 |
| (1-12) | -F | -CH₃ | (1-32) | -F | -CH₂CH₃ | (1-52) | -F | 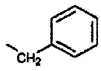 |
| (1-13) | -Cl | -CH₃ | (1-33) | -Cl | -CH₂CH₃ | (1-53) | -Cl | 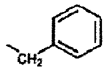 |
| (1-14) | -Br | -CH₃ | (1-34) | -Br | -CH₂CH₃ | (1-54) | -Br | 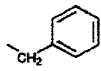 |
| (1-15) | -1 | -CH₃ | (1-35) | -I | -CH₂CH₃ | (1-55) | -I | 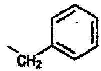 |
| (1-16) | -(CH₂)₃CH₃ | -CH₃ | (1-36) | -(CH₂)₃CH₃ | -CH₂CH₃ | (1-56) | -(CH₂)₃CH₃ | 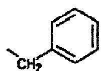 |

**Table 2**

| | | | | | | | | |
|---|---|---|---|---|---|---|---|---|
| 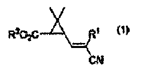 | | | | | | | | |

| Number | R¹ | R² | Number | R¹ | R² | Number | R¹ | R² |
|---|---|---|---|---|---|---|---|---|
| (1-61) | -CH₃ | 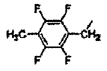 | (1-81) | -CH₃ | 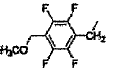 | (1-101) | -CH₃ | 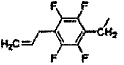 |
| (1-62) | -CH₂CH₃ | 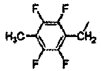 | (1-82) | -CH₂CH₃ | 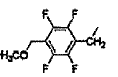 | (1-102) | -CH₂CH₃ | 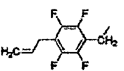 |
| (1-63) | -CH₂CH₂CH₃ | 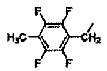 | (1-83) | -CH₂CH₂C₃ | 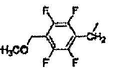 | (1-103) | -CH₂CH₂C₃ | 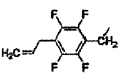 |
| (1-64) | 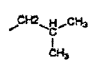 | 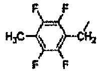 | (1-94) | 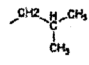 | 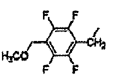 | (1-104) | 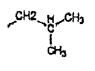 | 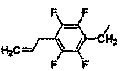 |
| (1-65) | 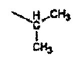 | 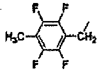 | (1-85) | 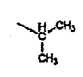 | 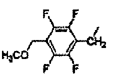 | (1-105) | 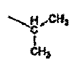 | 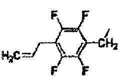 |
| (1-66) | 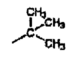 | 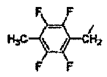 | (1-88) | 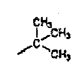 | 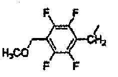 | (1-100) | 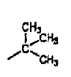 | 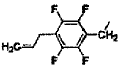 |
| (1-67) | -(CH₂)₄CH₃ | 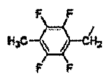 | (1-87) | -(CH₂)₄CH₃ | 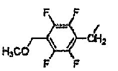 | (1-107) | -(CH₂)₄CH₃ | 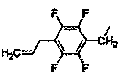 |
| (1-68) | 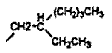 | 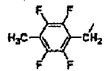 | (1-68) | 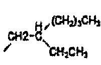 | 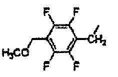 | (1-108) | 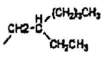 | 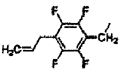 |
| | 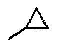 | 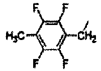 | (1-89) | 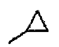 | 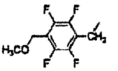 | (1-109) | 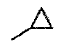 | 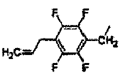 |
| (1-70) | 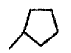 | 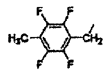 | (1-90) | 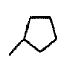 | 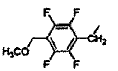 | (1-110) | 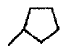 | 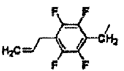 |
| (1-71) | 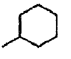 | 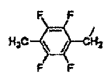 | (1-91) | 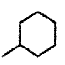 | | (1-111) | 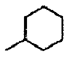 | 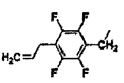 |
| (1-72) | -F | 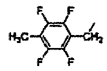 | (1-92) | -F | 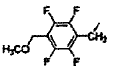 | (1-112) | -F | 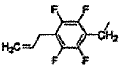 |
| (1-73) | -Cl | 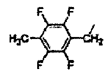 | (1-93) | -Cl | 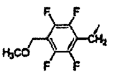 | (1-113) | -Cl | 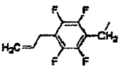 |
| (1-74) | -Br | 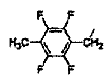 | (1-94) | -Br | 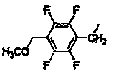 | (1-114) | -Br | 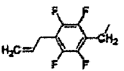 |
| (1-75) | -I | 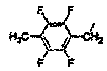 | (1-95) | -I | 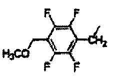 | (1-115) | -I | 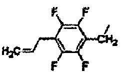 |
| (1-76) | -(CH₂)₃CH₃ | 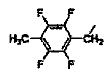 | (1-96) | -(CH₂)₃CH₃ | 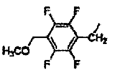 | (1-116) | -(CH₂)₃CH₂ | 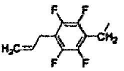 |

**Table 3**

| | | | | | | | | |
|---|---|---|---|---|---|---|---|---|
| 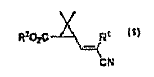 | | | | | | | | |

| Number | R¹ | R² | Number | R¹ | R² | Number | R² | R² |
|---|---|---|---|---|---|---|---|---|
| (1-121) | -CH₃ | 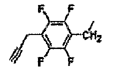 | (1-141) | -CH₃ | 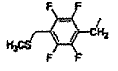 | (1-181) | -CH₃ | -H |
| (1-122) | -CH₂CH₃ | 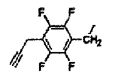 | (1-142) | -CH₂CH₃ | 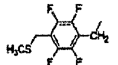 | (1-162) | -CH₂CH₃ | -H |
| (1-123) | -CH₂CH₂CH₃ | 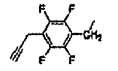 | (1-143) | -CH₂CH₂CH₃ | 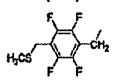 | (1-183) | -CH₂CH₂CH₃ | -H |
| (1-124) | 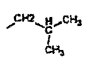 | 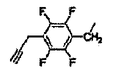 | (1-144) | 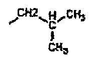 | 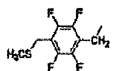 | (1-164) | 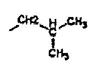 | -H |
| (1-125) | 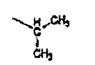 | 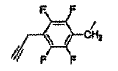 | (1-145) | 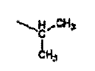 | 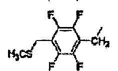 | (1-165) | 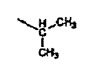 | -H |
| (1-126) | 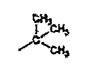 | 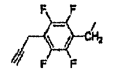 | (1-145) | 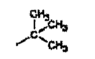 | 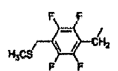 | (1-166) | 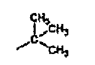 | -H |
| (1-127) | -(CH₂)₄CH₃ | 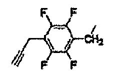 | (1-147) | -(CH₂)₄CH3 | 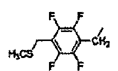 | (1-167) | -(CH₂)₄CH₃ | -H |
| (1-128) | 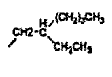 | 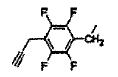 | (1-148) | 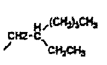 | 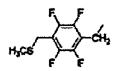 | (1-168) | 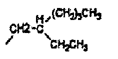 | -H |
| (1-129) | 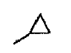 | 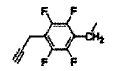 | (1-149) | 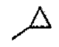 | 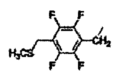 | (1-169) | 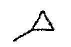 | -H |
| (1-130) | 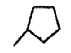 | 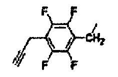 | (1-150) | 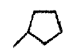 | 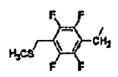 | (1-170) | 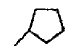 | -H |
| (1-131) | 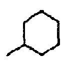 | 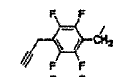 | (1-151) | 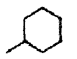 | 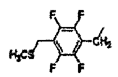 | (1-171) | 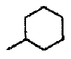 | -H |
| (1-132) | -F | 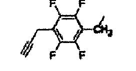 | (1-152) | -F | 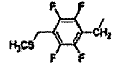 | (1-172) | -F | -H |
| (1-133) | -Cl | 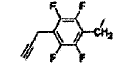 | (1-153) | -Cl | 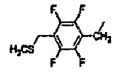 | (1-173) | -Cl | -H |
| (1-134) | -Br | 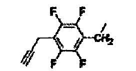 | (1-154) | -Br | 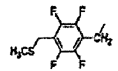 | (1-174) | -Br | -H |
| (1-135) | -I | 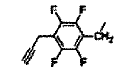 | (1-155) | -I | 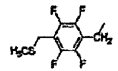 | (1-175) | -I | -H |
| (1-136) | -(CH₂)₃CH₃ | 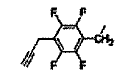 | (1-156) | -(CH₂)₃CH₃ | 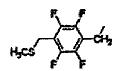 | (1-176) | -(CH₂)₃CH₃ | -H |

Preferably, the compound (1) is a compound indicated by the number (1-1), (1-13), (1-21), (1-33), (1-61), (1-73), (1-81), (1-93), (1-161), (1-162), (1-163), (1-164), (1-165), (1-166), (1-173) or (1-174) in Table 1, Table 2 and Table 3, and particularly preferably a compound indicated by the number (1-1), (1-21), (1-61), (1-81), (1-161) or (1-173) in Table 1, Table 2 and Table 3.

The compound (1) used in the isomerization step may be a mixture with the compound (2). When a mixture of the compound (1) and the compound (2) is used in the isomerization step as the compound (1), the content of the compound (1) is usually 30% by weight or more and less than 100% by weight based on the total amount of the compounds (1) and (2).

For example, the method described in Agr. Biol. Chem., 34, 1119 (1970) is provided as an example of preparing the compound (1). Specific examples thereof include a method in which a cyclopropane aldehyde represented by formula (3) : 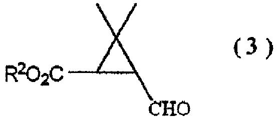 wherein R² has the same meaning as defined above, is reacted with a phosphonate compound represented by formula (4): 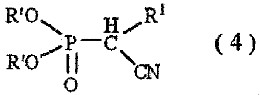 wherein R¹ has the same meaning as defined above, and R' represents an alkyl group having 1 to 4 carbon atoms, such as a methyl group or an ethyl group,
and a method in which a compound obtained by the method is subjected to ester hydrolysis.

The ester hydrolysis can be carried out, for example, by an acid, such as p-toluenesulfonic acid, or an aqueous solution of an alkali metal hydroxide, such as sodium hydroxide or potassium hydroxide.

Examples of a method for preparing the compound (1) include the method described in JP-A-2008-44900. Specific examples thereof include a method in which a cyclopropane aldehyde represented by formula (3) is reacted with an aldehyde compound represented by formula (5): 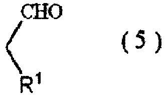 wherein R¹ has the same meaning as defined above, in the presence of a base, the obtained unsaturated aldehyde compound represented by formula (6): 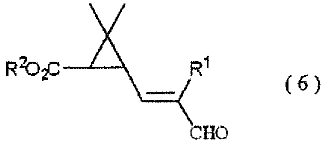 wherein R¹ and R² are as defined above,
is further reacted with hydroxylamine or a hydrochloride or a sulfate thereof or the like, and the obtained oxime compound represented by formula (7): 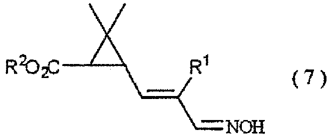 wherein R¹ and R² are as defined above, is reacted with a dehydrating agent in the presence of a base, and a method in which a compound obtained by the above method is subjected to ester hydrolysis. The ester hydrolysis can be carried out, for example, using an acid, such as p-toluenesulfonic acid, or using an aqueous solution of an alkali metal hydroxide, such as sodium hydroxide and potassium hydroxide.

The isomerizing catalyst used in the isomerization step has a capability of isomerizing the compound (1) into the compound (2). Specific examples of the isomerizing catalyst include:
bromine;
hydrogen bromide;
brominated carboxylic acids, such as acetyl bromide, propionyl bromide and benzoyl bromide;
brominated phosphorus compounds, such as phosphorus tribromide and phosphorus pentabromide;
N-brominated imide compounds, such as N-bromosuccinimide; N-brominated amide compounds, such as N-bromoacetamide; brominated alkylsilane compounds, such as trimethylsilyl bromide and silicon tetrabromide;
thionyl bromide;
brominated boron compounds, such as boron tribromide; brominated aluminum compounds, such as aluminum tribromide; thiol compounds, such as thiophenol, p-thiocresol and 1-butanethiol;
disulfide compounds, such as diphenyl disulfide; thiocarboxylic acid compounds, such as thiobenzoic acid, thioacetic acid and thiosalicylic acid;
nitric acid; and
nitrate salts, such as sodium nitrate.

Preferably, examples of the isomerizing catalyst include bromine, hydrogen bromide, brominated phosphorus compounds, and disulfide compounds, and more preferably include bromine, hydrogen bromide, phosphorus tribromide, and diphenyl sulfide. When the compound (1) is a compound in which R² in formula (1) is an alkyl group which may have a substituent, or a benzyl group which may have a substituent, bromine, hydrogen bromide and phosphorus tribromide are more preferable as an isomerizing catalyst. When the compound (1) is a compound in which R² in formula (1) is hydrogen, hydrogen bromide and diphenyl sulfide are more preferable as an isomerizing catalyst.

The use amount of the isomerizing catalyst is, for example, within a range of from 0.01 to 0.5 mol, and preferably from 0.05 to 0.2 mol, based on 1 mol of the compound (1).

When hydrogen bromide is used as an isomerizing catalyst, the hydrogen bromide is preferably used in the form of an acetic acid solution containing hydrogen bromide or an aqueous hydrobromic acid solution. When an aqueous hydrobromic acid solution is used as an isomerizing catalyst and an organic solvent not compatible with water (for example, an aromatic hydrocarbon, a halogenated aromatic hydrocarbon, an aliphatic hydrocarbon, an alicyclic hydrocarbon, etc.) is used as a solvent mentioned below, the isomerization can proceed more smoothly in the presence of an inorganic salt, which has high solubility in water and does not inhibit a reaction, in the reaction system. Specific examples of the inorganic salt include lithium bromide, lithium chloride, calcium bromide, calcium chloride, magnesium bromide, magnesium chloride and magnesium sulfate.

The isomerization step is preferably carried out Further in the presence of a radical initiator. The radical initiator is a compound different from the isomerizing catalyst and means a compound which can generate a radical.

Examples of the radical initiator include:
inorganic peroxides, such as ammonium persulfate, potassium persulfate, sodium persulfate and hydrogen peroxide;
ketone peroxide compounds, such as methyl ethyl ketone peroxide and cyclohexanone peroxide;
hydroperoxide compounds, such as t-butyl hydroperoxide, 1,1,3,3-tetramethylbutyl hydroperoxide, cumene hydroperoxide and triphenylmethyl peroxide;
diacyl peroxide compounds, such as benzoyl peroxide (dibenzoyl peroxide) and diacetyl peroxide;
peracid ester compounds, such as t-butyl perbenzoate and t-butyl peracetate;
dialkyl peroxides, such as di-t-butyl peroxide;
peracid compounds, such as peracetic acid, perbenzoic acid and m-chlorobenzoic acid; and
azo compounds, such as azobisisobutyronitrile, 2,2'-azobis(4-methoxy-2,4-dimethylvaleronitrile), 2,2'-azobis(2,4-dimethylvaleronitrile), 1,1'-azobis(cyclohexane-1-carbonitrile), 4,4'-azobis(4-cyanopentanoic acid), 1-[(1-cyano-l-methylethyl)azo)formamide, 2,2'-azobis(2-amidinopropane), 2,2'-azobis[2-methyl-N-(2-hydroxyethyl)propionamide) and methyl azobisisobutylate.

Preferably, examples of the radical initiator include hydroperoxide compounds, diacyl peroxide compounds and azo compounds, and more preferably include t-butyl hydroperoxide, benzoyl peroxide, azobisisobutyronitrile, and 2,2'-azobis(4-methoxy-2,4-dimethylvaleronitrile).

The use amount of the radical initiator is usually within a range of from 0 to 0.3 mol and preferably from 0.01 to 0.1 mol, based on 1 mol of the compound (1).

The reaction temperature in the isomerization step is usually within a range of from 0 to 80°C, and preferably from 20 to 60°C. When the isomerization step is carried out in the presence of a radical initiator, it is also permitted to heat the reaction mixture, as needed, to a temperature equal to or higher than the temperature at which the radical initiator generates a radical to start the isomerization of the compound (1) to the compound (2), and then adjust the temperature of the mixture to a reaction temperature within the above range.

The reaction time in the isomerization step is usually within a range of from 1 minute to 24 hours, and preferably from 1 hour to 10 hours.

The isomerization step can be carried out in the absence of any solvent, providing that the reaction temperature in the isomerization step is a temperature equal to or higher than the melting points of the compound (1) and the compound (2). However, the isomerization step is preferably carried out in the presence of a solvent regardless of the reaction temperature of the isomerization step. Examples of the solvent to be used include:
aromatic hydrocarbons, such as toluene, xylene and ethylbenzene;
aliphatic hydrocarbons, such as n-pentane and n-hexane;
alicyclic hydrocarbons, such as cyclopentane and cyclohexane;
halogenated aromatic hydrocarbons, such as monochlorobenzene; and
ether compounds, such as diethyl ether, tert-butyl methyl ether, 1,2-dimethoxyethane, 1,4-dioxane, tetrahydrofuran and anisole.

The use amount of the solvent is usually within a range of from 0.50 to 20 parts by weight based on 1 part by weight of the compound (1).

The isomerization step is carried out, for example, by:
(A) a method in which an isomerizing catalyst and a radical initiator as needed are gradually added to a solution containing the compound (1) and a solvent at a reaction temperature, and the obtained mixture is then stirred; or
(B) a method in which an isomerizing catalyst and a radical initiator as needed are added to a solution containing the compound (1) and a solvent, and then the obtained mixture is adjusted to a reaction temperature and stirred.
When a radical initiator is used, the whole or a part of a mixture containing the compound (1), a solvent, an isomerizing catalyst and a radical initiator may be heated to a temperature equal to or higher than the temperature at which the radical initiator generates a radical. After heating, the mixture containing the compound (1), a solvent, an isomerizing catalyst and a radical initiator may be adjusted to a reaction temperature.

After completion of the isomerization step, the obtained reaction mixture may be subjected, for example, to a post-treatment, such as neutralization, extraction and washing with water, and a mixture after such a post-treatment may be subjected further to a purification treatment. Examples of the purification treatment include purification by crystallization, purification by extraction, purification by distillation, purification by adsorption by activated carbon, purification by adsorption by silica, purification by adsorption by alumina, and purification by chromatography, such as silica gel column chromatography.

The compound (2) is obtained by the isomerization step.

The compound (2) is represented by formula (2). R¹ and R² in formula (2) have the same meanings as those of R¹ and R² in formula (1), respectively. With respect to R¹ and R² in formula (2), a preferred range, a more preferred range and a still more preferred range are the same as those in formula (1), respectively.

The compound (2) obtained through the isomerization step may be a mixture with the compound (1). In this case, the content of the compound (2) is usually from 1 to 80% by weight, more preferably from 30 to 80% by weight, and still more preferably from 50 to 70% by weight, based on the total amount of the compounds (1) and (2). When the compound (1) used in the isomerization step is a mixture with the compound (2), the content of the compound (2) based on the total amount of the compounds (1) and (2) increases by passing through the isomerization step.

In the compound (2) obtained via the isomerization step, the steric configuration of an asymmetric carbon atom contained in its cyclopropane ring is retained in almost the same manner as the steric configuration of an asymmetric carbon atom contained in the cyclopropane ring in the compound (1) used in the isomerization step. For example, when explaining this using methyl (E)-2,2-dimethyl-3-[2-cyano-1-propen-1-yl)cyclopropanecarboxylate (hereinafter sometimes referred to as compound (1-1)), the compound (1-1) in which both position 1 and position 3 of the cyclopropane ring are in R configuration (1R, 3R) provides, via the isomerization step, methyl (Z)-2,2-dimethyl-3-[2-cyano-1-propen-1-yl)cyclopropanecarboxylate (hereinafter sometimes referred to as compound (2-1)) in which both position 1 and position 3 of the cyclopropane ring are retained in R configuration (1R, 3R), and the optical purity of the compound (2-1) obtained through the isomerization step is almost identical with the optical purity of the compound (1-1) used in the isomerization step.

Examples of the compound (2) thus obtained include compounds indicated by the numbers (2-1) to (2-176) shown in Table 4, Table 5 and Table 6.

**Table 4**

| | | | | | | | | |
|---|---|---|---|---|---|---|---|---|
| 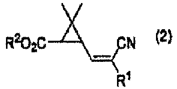 | | | | | | | | |

| Number | R¹ | R² | Number | R¹ | R² | Number | R¹ | R² |
|---|---|---|---|---|---|---|---|---|
| (2-1) | -CH₃ | -CH₃ | (2-21) | -CH₃ | -CH₂CH₃ | (2-41) | -CH₃ | 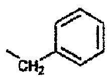 |
| (2-2) | -CH₂CH₃ | -CH₃ | (2-22) | -CH₂CH₃ | -CH₂CH₃ | (2-42) | -CH₂CH₃ | 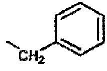 |
| (2-3) | -CH₂CH₂CH₃ | -CH₃ | (2-23) | -CH₂CH₂CH₃ | -CH₂CH₃ | (2-43) | -CH₂CH₂CH₃ | 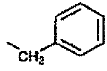 |
| (2-4) | 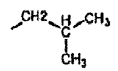 | -CH₃ | (2-24) | 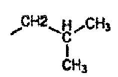 | -CH₂CH₃ | (2-44) | 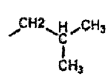 | 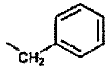 |
| (2-5) | 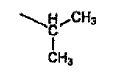 | -CH₃ | (2-25) | 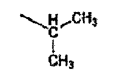 | -CH₂CH₃ | (2-45) | 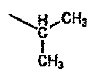 | 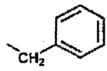 |
| (2-6) | 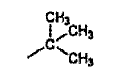 | -CH₃ | (2-26) | 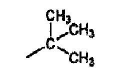 | -CH₂CH₃ | (2-46) | 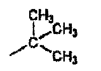 | 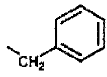 |
| (2-7) | -(CH₂)₄CH₃ | -CH₃ | (2-27) | -(CH₂)₄CH₃ | -CH₂CH₃ | (2-47) | -(CH₂)₄CH₃ | 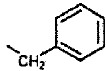 |
| (2-8) | 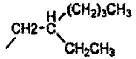 | -CH₃ | (2-28) | 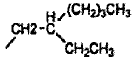 | -CH₂CH₃ | (2-46) | 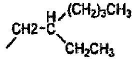 | 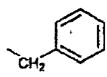 |
| (2-9) | 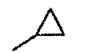 | -CH₃ | (2-29) | 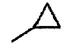 | -CH₂CH₃ | (2-49) | 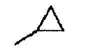 | 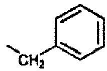 |
| (2-10) | 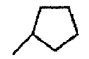 | -CH₃ | (2-30) | 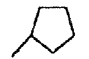 | -CH₂CH₃ | (2-50) | 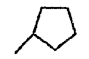 | 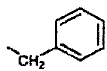 |
| (2-11) | 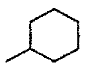 | -CH₃ | (2-31) | 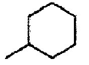 | -CH₂CH, | (2-51) | 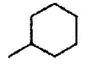 | 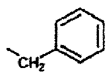 |
| (2-12) | -F | -CH₃ | (2-32) | -F | -CH₂CH₃ | (2-52) | -F | 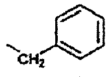 |
| (2-13) | -Cl | -CH₃ | (2-33) | -Cl | -CH₂CH₃ | (2-53) | -Cl | 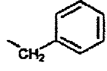 |
| (2-14) | -Br | -CH₃ | (2-34) | -Br | -CH₂CH₃ | (2-54) | -Br | 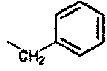 |
| (2-15) | -I | -CH₃ | (2-35) | -I | -CH₂CH₃ | (2-55) | -I | 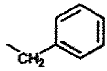 |
| (2-16) | -(CH₂)₃CH₃ | -CH₃ | (2-36) | -(CH₂)₃CH₃ | -CH₂CH₃ | (2-56) | -(CH₂)₃CH₃ | 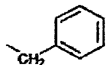 |

**Table 5**

| | | | | | | | | |
|---|---|---|---|---|---|---|---|---|
| 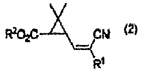 | | | | | | | | |

| Number | R¹ | R² | Number | R¹ | R² | Number | R¹ | R² |
|---|---|---|---|---|---|---|---|---|
| (2-61) | -CH₃ | 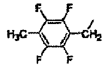 | (2-81) | -CH₃ | 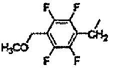 | (2-101) | -CH₃ | 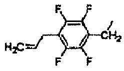 |
| (2-82) | -CH₂CH₃ | 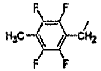 | (2-82) | -CH₂CH₃ | 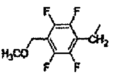 | (2-102) | -CH₂CH₃ | 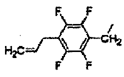 |
| (2-63) | -CH₂CH₂CH₃ | 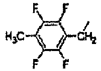 | (2-83) | -CH₂CH₂CH₃ | 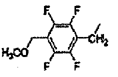 | (2-103) | -CH₂CH₂CH₃ | 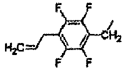 |
| (2-64) | 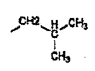 | 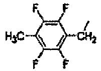 | (2-84) | 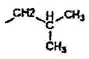 | 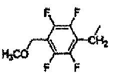 | (2-104) | 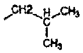 | 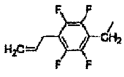 |
| (2-65) | 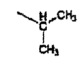 | 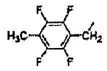 | (2-85) | 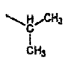 | 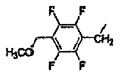 | (2-105) | 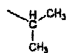 | 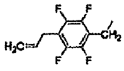 |
| (2-66) | 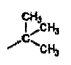 | 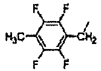 | (2-86) | 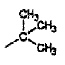 | 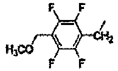 | (2-106) | 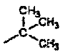 | 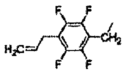 |
| (2-67) | -(CH₂)₄CH₃ | 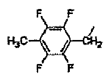 | (2-87) | -(CH₂)₄CH₃ | 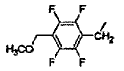 | (2-107) | -(CH₂)₄CH₃ | 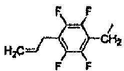 |
| (2-68) | 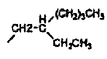 | 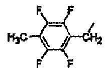 | (2-88) | 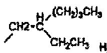 | 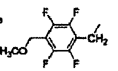 | (2-108) | 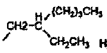 | 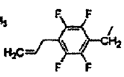 |
| (2-69) | 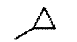 | 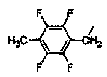 | (2-89) | 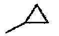 | 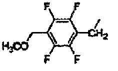 | (2-109) | 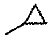 | 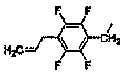 |
| (2-70) | 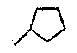 | 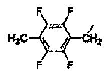 | (2-90) | 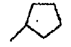 | 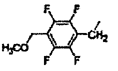 | (2-110) | 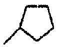 | 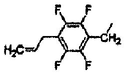 |
| (2-71) | 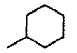 | 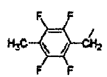 | (2-91) | 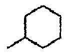 | 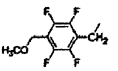 | (2-111) | 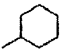 | 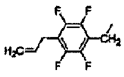 |
| (1-72) | -F | 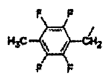 | (2-92) | -F | 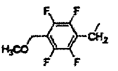 | (2-112) | -F | 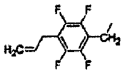 |
| (2-73) | -Cl | 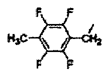 | (2-93) | -Cl | 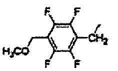 | (2-113) | -Cl | 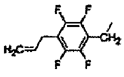 |
| (2-74) | -Br | 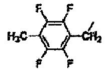 | (2-94) | -Br | 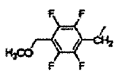 | (2-114) | -Br | 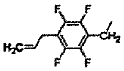 |
| (2-75) | -I | 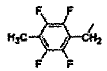 | (2-95) | -I | 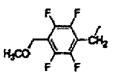 | (2-115) | -I | 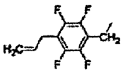 |
| (2-76) | -(CH₂)₂CH₃ | 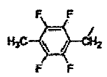 | (2-96) | -(CH₂)₃CH₃ | 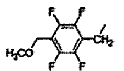 | (2-116) | -(CH₂)₃CH₃ | 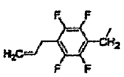 |

**Table 6**

| | | | | | | | | |
|---|---|---|---|---|---|---|---|---|
| 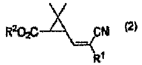 | | | | | | | | |

| | Number R¹ | R² | Number | R¹ | R² | Number | R¹ | R² |
|---|---|---|---|---|---|---|---|---|
| (2-121) | -CH₃ | 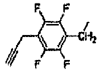 | (2-141) | -CH₃ | 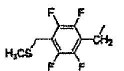 | (2-161) | -CH₃ | -H |
| (2-122) | -CH₂CH₃ | 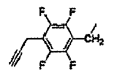 | (2-142) | -CH₂CH₃ | 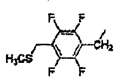 | (2-102) | -CH₂CH₃ | -H |
| (2-123) | -CH₂CH₂CH₃ | 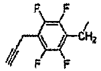 | (2-143) | -CH₂CH₂CH₃ | 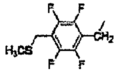 | (2-163) | -QH₂CH₂CH₃ | -H |
| (2-124) | 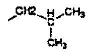 | 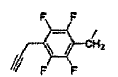 | (2-144) | 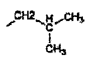 | 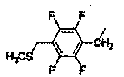 | (2-164) | 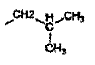 | -H |
| (2-125) | 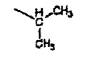 | 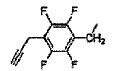 | (2-145) | 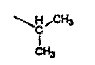 | 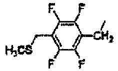 | (2-155) | 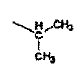 | -H |
| (2-126) | 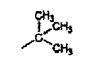 | 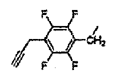 | (2-146) | 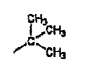 | 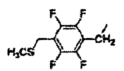 | (2-166) | 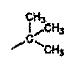 | -H |
| (2-127) | -(CH₂)₄CH₃ | 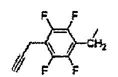 | (2-147) | -(CH₂)₄CH₃ | 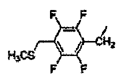 | (2-167) | -(CH₂)₄CH₃ | -H |
| (2-128) | 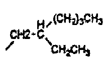 | 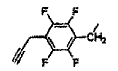 | (2-148) | 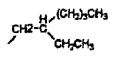 | 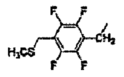 | (2-168) | 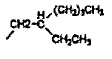 | -H |
| (2-128) | 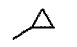 | 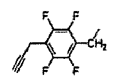 | (2-149) | 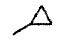 | 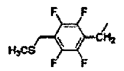 | (2-163) | 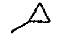 | -H |
| (2-130) | 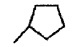 | 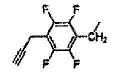 | (2-150) | 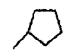 | 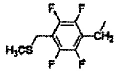 | (2-170) | 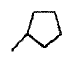 | -H |
| (2-131) | 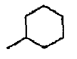 | 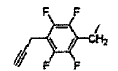 | (2-151) | 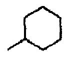 | 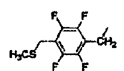 | (2-171) | 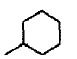 | -H |
| (2-132) | -F | 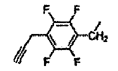 | (2-152) | -F | 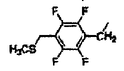 | (2-172) | -F | -H |
| (1-133) | -Cl | 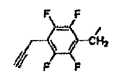 | (2-153) | -Cl | 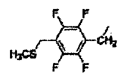 | (2-173) | -Cl | -H |
| (1-134) | -Br | 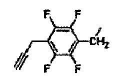 | (2-154) | -Br | 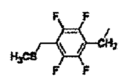 | (2-174) | -Br | -H |
| (2-135) | -I | 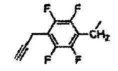 | (2-155) | -I | 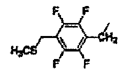 | (2-175) | -I | -H |
| (2-136) | -(CH₂)₃CH₃ | 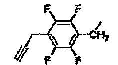 | (2-156) | -(CH₂)₂CH₃ | 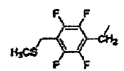 | (2-176) | -(CH₂)₂CH₃ | -H |

Preferably, the compound (2) is a compound indicated by the number (2-1), (2-13), (2-21), (2-33), (2-61), (2-73), (2-81), (2-93), (2-161), (2-162), (2-163), (2-164), (2-165), (2-166), (2-173) or (2-174) in Table 4, Table 5 and Table 6, and particularly preferably a compound indicated by the number (2-1), (2-21), (2-61), (2-81), (2-161) or (2-173) in Table 4, Table 5 and Table 6.

### EXAMPLES

The present invention will be described below by way of Examples. In the Examples, the content of each of the compound (1) and the compound (2) was determined by gas chromatography using an internal reference method, on the basis of the retention time in gas chromatography analysis of each of the compound (1) and the compound (2) separately prepared by recrystallization and the like.

### <Production Example 1> Production of compound (1)

Under a nitrogen atmosphere, an aqueous 24% by weight solution (123 g) of hydroxylamine sulfate, methanol (about 30 g) and 80% by weight acetic acid (11.27 g) were added to a xylene solution (130 g) of methyl 2,2-dimethyl-3-[2-formyl-1-propenyl)cyclopropanecarboxylate (concentration: 45.4% by weight), and then an aqueous 23% by weight solution (70.6 g) of sodium hydroxide was added dropwise to the mixture at 21°C over 13 hours while maintaining the pH at pH 5. Subsequently, the reaction mixture was separated, and the obtained oil layer was washed twice with water. Then, 35% by weight hydrochloric acid (6.20 g) and pyridine (24.0 g) were added to the obtained oil layer and the mixture was stirred for 30 minutes. Acetic anhydride (75.90 g) was added dropwise to the mixture at 20°C over 3 hours, and then the reaction solution was heated to 80°C and stirred for 14 hours. After cooling, xylene (59.0 g) and water (276 g) were added to the reaction mixture and 98% by weight sulfuric acid (14.8 g) was added dropwise to the mixture, and then the mixture was separated. The obtained aqueous layer was extracted with xylene and the xylene layer was combined with the oil layer obtained previously, and then the combined layer was sequentially washed with an aqueous 7% by weight solution of sodium carbonate and water. Subsequently, the oil layer was concentrated under a pressure of 3.0 kPa to obtain 66.9 g of methyl 2,2-dimethyl-3-(2-cyano-1-propenyl)-cyclopropanecarboxylate.

### Concentration: 76.1% (gas chromatography using an internal reference method)

### Yield: 88%

Analysis of the obtained methyl 2,2-dimethyl-3-(2-cyano-1-propenyl)cyclopropanecarboxylate by gas chromatography revealed that the content of methyl (Z)-2,2-dimethyl-3-(2-cyano-1-propenyl)-cyclopropanecarboxylate (hereinafter sometimes referred to as compound (2-1)) was 1.9% by weight based on the total amount of methyl (E)-2,2-dimethyl-3-(2-cyano-1-propenyl)-cyclopropanecarboxylate (hereinafter sometimes referred to as compound (1-1)) and the compound (2-1).

### <Production Example 2> Production of compound (1)

Under a nitrogen atmosphere, heptane (74.2 g) was added to methyl 2,2-dimethyl-3-(2-cyano-1-propenyl)-cyclopropanecarboxylate (65.0 g) obtained in the same manner as in Production Example 1, and the compound was dissolved at 57°C. The obtained solution was cooled to 38°C over 45 minutes and a seed crystal was added to the solution at the same temperature, and then the mixture was cooled to 33°C. The cooled mixture was stirred for 1 hour at the same temperature and was further cooled to 1.3°C over 3 hours. The obtained mixture was stirred for 1 hour at the same temperature and then filtrated under reduced pressure. Washing of a wet cake with heptane cooled to about 0°C was repeated twice. The cake was dried under reduce pressure to obtain 51.9 g of purified methyl 2,2-dimethyl-3-(2-cyano-1-propenyl)-cyclopropanecarboxylat**e.**

### Purity: 92.1% (gas chromatography using an internal reference method)

### Recovery rate: 95%

Under a nitrogen atmosphere, methanol (44.2 g) and water (88.5 g) were added to the above-mentioned methyl 2,2-dimethyl-3-(2-cyano-1-propenyl)-cyclopropanecarboxylate (50.6 g), and the obtained mixture was warmed to 54°C. An aqueous 23% by weight solution (41.85 g) of sodium hydroxide was added dropwise to the mixture over 1 hour, and the mixture was stirred for 3 hours at the same temperature. Subsequently, an aqueous 23% by weight solution (2.00 g) of sodium hydroxide was further added to the mixture, and the mixture was stirred for 1 hour. After the obtained mixture was cooled to 20°C, toluene and water were added to the mixture, and then 98% by weight sulfuric acid (13.3 g) was added dropwise to the mixture and the mixture was separated. Extraction was carried out by adding toluene to the obtained aqueous layer, the toluene layer was combined with the oil layer previously obtained, and then the combined layer was washed with water. Subsequently, the oil layer was partially concentrated under a pressure of 30 kPa to obtain a toluene solution (130 g) containing (E)-2,2-dimethyl-3-(2-cyano-1-propenyl)-cyclopropanecarboxylic acid (hereinafter sometimes referred to as compound (1-161)) as a main component and also containing (Z)-2,2-dimethyl-3-(2-cyano-1-propenyl)-cyclopropanecarboxylic acid (hereinafter sometimes referred to as compound (2-161)).
Moisture value: 216 ppm (Karl Fischer coulometric titration method)
Total yield of compounds (1-161) and (2-161): 95.4% Total concentration of compounds (1-161) and (2-161): 39.8%
Compound (1-161): compound (2-161) = 98.9 : 1.1 (ratio by weight)
Isomer ratio based on 100% by weight in total of compounds (1-161) and (2-161):
   (1R, 3R)-isomer: 97.5%
   (1R, 3S)-isomer: 0.9%
   (1S, 3R)-isomer: 0.0%
   (1S, 3S)-isomer: 1.6%

### <Production Example 3>

Under a nitrogen atmosphere, an aqueous 24% by weight solution (19.7 g) of hydroxylamine sulfate and octanoic acid (7.9 g) were added to a xylene solution (224.1 g) of methyl 2,2-dimethyl-3-[2-formyl-1-propenyl)-cyclopropanecarboxylate (concentration: 48.1% by weight). To the mixture, an aqueous 24% by weight solution (196.6 g) of hydroxylamine sulfate and an aqueous 23% by weight solution (124.4 g) of sodium hydroxide were respectively added dropwise at 25°C over 7 hours while maintaining the pH at pH 7 or below, and then the mixture was maintained at the same temperature for 2 hours. Subsequently, the reaction mixture was separated, and the oil layer was washed twice with an aqueous 10% by weight solution (107.9 g) of sodium sulfate. The obtained oil layer was subjected to pressure reduction under a pressure of 5 kPa and dehydrated with heating under reflux to obtain a mixed xylene solution (239.1 g) of methyl 2,2-dimethyl-3-[3-(hydroxyimino)-2-methyl-1-propenyl)-cyclopropanecarboxylate.

### Concentration: 48.4% (gas chromatography using an internal reference method)

### Yield: 99.6%

Under a nitrogen atmosphere, pyridine (43.5 g), mixed xylene (105.6 g), pyridine hydrochloride (11.6 g) and acetic anhydride (2.6 g) were mixed, and the mixture was heated to 100°C. To the mixture, the mixed xylene solution (218.5 g) of methyl 2,2-dimethyl-3-[3-(hydroxyimino)-2-methyl-1-propenyl)-cyclopropanecarboxylate (concentration: 48.4% by weight) obtained in the above example and acetic anhydride (63.8 g) were added dropwise at the same temperature over 6 hours, and then the mixture was maintained at the same temperature for 2 hours. After cooling, water (105.6 g) was added to the reaction mixture and 98% by weight sulfuric acid (33.4 g) was added dropwise to the mixture, and then the mixture was separated. To the obtained oil layer, water (105.6 g) was added, and then an aqueous 23% by weight solution (60.9 g) of sodium hydroxide was added dropwise and the mixture was separated. After washing with a 10% aqueous solution (105.6 g) of sodium sulfate, the oil layer was concentrated under a pressure of 5.0 kPa to obtain a mixed xylene solution (161.6 g) of methyl 2,2-dimethyl-3-(2-cyano-1-propenyl)-cyclopropanecarboxylate.

### Concentration: 58.5% (gas chromatography using an internal reference method)

### Yield: 97.8%

Analysis of the obtained methyl 2,2-dimethyl-3-(2-cyano-1-propenyl)cyclopropanecarboxylate by gas chromatography revealed that the content of methyl (Z)-2,2-dimethyl-3-(2-cyano-1-propenyl)-cyclopropanecarboxylate (hereinafter sometimes referred to as compound (2-1)) was 1.6% by weight based on the total amount of methyl (E)-2,2-dimethyl-3-(2-cyano-1-propenyl)-cyclopropanecarboxylate (hereinafter sometimes referred to as compound (1-1)) and the compound (2-1).

### <Production Example 4> Production of compound (1)

Under a nitrogen atmosphere, methanol (86.6 g) and water (173.2 g) were added to methyl 2,2-dimethyl-3-(2-cyano-1-propenyl)-cyclopropanecarboxylate (148.0 g) obtained in the same manner as in Production Example 3, and the obtained mixture was warmed to 55°C. An aqueous 23% by weight solution (85.4 g) of sodium hydroxide was added dropwise to the mixture over 3 hours, and then the mixture was stirred for 2 hours at the same temperature. After the obtained mixture was cooled to 20°C, xylene (43.3 g) was added to the mixture, and the mixture was separated. To the obtained aqueous layer, xylene (86.6 g) and water (58.0 g) were added and 98% by weight sulfuric acid (26.9 g) was added dropwise, and then the mixture was separated. After the obtained aqueous layer was concentrated under a pressure of 20 kPa, extraction was carried out by adding xylene (86.6 g) to the layer. The xylene layer was combined with the oil layer previously obtained, and the combined layer was washed with water (43.3 g) to obtain a xylene solution (263.7 g) containing (E)-2,2-dimethyl-3-(2-cyano-1-propenyl)-cyclopropanecarboxylic acid (hereinafter sometimes referred to as compound (1-161)) as a main component and also containing (Z)-2,2-dimethyl-3-(2-cyano-1-propenyl)-cyclopropanecarboxylic acid (hereinafter sometimes referred to as compound (2-161)).

Total yield of compounds (1-161) and (2-161): 100.1%
Total concentration of compounds (1-161) and (2-161): 30.5%
Under a nitrogen atmosphere, 619.6 g (concentration: 24.1% by weight) of 2,2-dimethyl-3-(2-cyano-1-propenyl)-cyclopropanecarboxylic acid obtained by the above method was concentrated under a pressure of 10 kPa to obtain 267.0 g (concentration: 55.7% by weight) of 2,2-dimethyl-3-(2-cyano-1-propenyl)-cyclopropanecarboxylic acid. Heptane (122.9 g) was added to the obtained oil layer, and the compound was dissolved at 69°C. The obtained solution was cooled to 58°C over 1.5 hours, a seed crystal was added to the solution at the same temperature, and then the mixture was maintained at the same temperature for 2 hours. The mixture was further cooled to 55°C over 2 hours, then cooled to 2°C over 5 hours, and maintained at the same temperature. The obtained mixture was filtrated under reduced pressure, and the solid was sequentially washed with a heptane solution (149.0 g) containing xylene in a proportion of 45% by weight and heptane (149.0 g) which were cooled to about 0°C. The solid was dried under reduce pressure to obtain 140.9 g of purified 2,2-dimethyl-3-(2-cyano-1-propenyl)-cyclopropanecarboxylic acid.

Total yield of compounds (1-161) and (2-161): 93.6%
Total concentration of compounds (1-161) and (2-161): 99.0%
Compound (1-161): compound (2-161) = 98.9 : 1.1 (ratio by weight)
Isomer ratio based on 100% by weight in total of compounds (1-161) and (2-161):
   (1R, 3R)-isomer. 95.4%
   (1R, 3S)-isomer: 1.7%
   (1S, 3R)-isomer: 0.0%
   (1S, 3S)-isomer: 2.9%

### <Isomerization step>

### (Example 1)

Under a nitrogen atmosphere, a toluene solution (about 40 mg) containing 50% by weight of t-butyl hydroperoxide and bromine (about 100 mg) were added to a mixture of methyl 2,2-dimethyl-3-(2-cyano-1-propenyl)-cyclopropanecarboxylate (1.0 g) obtained in Production Example 1 and heptane (51.9 g). A part of the obtained mixture was extracted and heated with a drier to initiate the isomerization of the compound (1-1) into the compound (2-1). Subsequently, the part was returned again to the mixture, and the mixture was stirred at 24°C for 1 hour.

Analysis of the obtained mixture (reaction mixture) by gas chromatography revealed that the content of the compound (2-1) was 57.7% by weight, based on the total amount of the compounds (1-1) and (2-1).

### (Example 2)

Under a nitrogen atmosphere, benzoyl peroxide (36.5 mg) and bromine (about 60 mg) were added to a mixture of methyl 2,2-dimethyl-3-(2-cyano-1-propenyl)-cyclopropanecarboxylate (1.0 g) obtained in Production Example 1 and heptane (48.7 g). A part of the obtained mixture was removed and heated with a drier to initiate the isomerization of the compound (1-1) into the compound (2-1). Subsequently, the part was returned again to the mixture, and the mixture was stirred at 24°C for 1 hour.

Analysis of the obtained mixture (reaction mixture) by gas chromatography revealed that the content of the compound (2-1) was 54.4% by weight based on the total amount of the compounds (1-1) and (2-1).

### (Example 3)

Under a nitrogen atmosphere, azobisisobutyronitrile (18.5 mg) and bromine (about 40 mg) were added to a mixture of methyl 2,2-dimethyl-3-(2-cyano-1-propenyl)-cyclopropanecarboxylate (1.0 g) obtained in Production Example 1 and heptane (48.8 g). A part of the obtained mixture was removed and heated with a drier to initiate the isomerization of the compound (1-1) into the compound (2-1). Subsequently, the part was returned again to the mixture, and the mixture was stirred at 24°C for 1 hour.

Analysis of the obtained mixture (reaction mixture) by gas chromatography revealed that the content of the compound (2-1) was 47.9% by weight, based on the total amount of the compounds (1-1) and (2-1).

### (Example 4)

Under a nitrogen atmosphere, 2,2'-azobis(4-methoxy-2,4-dimethylvaleronitrile) (73.7 mg) and bromine (61 mg) were added to a mixture of methyl 2,2-dimethyl-3-(2-cyano-1-propenyl)-cyclopropanecarboxylate (1.0 g) obtained in Production Example 1 and heptane (48.8 g). Subsequently, the mixture was stirred at the same temperature (20°C) for 1 hour.

Analysis of the obtained mixture (reaction mixture) by gas chromatography revealed that the content of the compound (2-1) was 58.1% by weight based on the total amount of the compounds (1-1) and (2-1).

### (Example 5)

Under a nitrogen atmosphere, a toluene solution (about 40 mg) containing 50% by weight of t-butyl hydroperoxide and phosphorus tribromide (102 mg) were added to a mixture of methyl 2,2-dimethyl-3-(2-cyano-1-propenyl)-cyclopropanecarboxylate (1.0 g) obtained in Production Example 1 and heptane (48.9 g). A part of the obtained mixture was removed and heated with a drier to initiate the isomerization of the compound (1-1) into the compound (2-1). Subsequently, the part was returned again to the mixture, and the mixture was stirred at 24°C for 1 hour.

Analysis of the obtained mixture (reaction mixture) by gas chromatography revealed that the content of the compound (2-1) was 10.6% by weight based on the total amount of the compounds (1-1) and (2-1).

### (Example 6)

Under a nitrogen atmosphere, benzoyl peroxide (34.8 mg) and phosphorus tribromide (99.8 mg) were added to a mixture of methyl 2,2-dimethyl-3-(2-cyano-1-propenyl)-cyclopropanecarboxylate (1.0 g) obtained in Production Example 1 and heptane (48.7 g). A part of the obtained mixture was removed and heated with a drier to initiate the isomerization of the compound (1-1) into the compound (2-1). Subsequently, the part was returned again to the mixture, and the mixture was stirred at 24°C for 1 hour.

Analysis of the obtained mixture (reaction mixture) by gas chromatography revealed that the content of the compound (2-1) was 15.9% by weight based on the total amount of the compounds (1-1) and (2-1).

### (Example 7)

Under a nitrogen atmosphere, a toluene solution (about 40 mg) containing 50% by weight of t-butyl hydroperoxide and an acetic acid solution (105 mg) containing 30% by weight of hydrobromic acid were added to a mixture of methyl 2,2-dimethyl-3-(2-cyano-1-propenyl)-cyclopropanecarboxylate (1.0 g) obtained in Production Example 1 and heptane (56.3 g). A part of the obtained mixture was removed and heated with a drier to initiate the isomerization of the compound (1-1) into the compound (2-1). Subsequently, the part was returned again to the mixture, and the mixture was stirred at 26°C for 1 hour.

Analysis of the obtained mixture (reaction mixture) by gas chromatography revealed that the content of the compound (2-1) was 12.6% by weight based on the total amount of the compounds (1-1) and (2-1).

### (Example 8)

Under a nitrogen atmosphere, benzoyl peroxide (34.3 mg) and an acetic acid solution (117 mg) containing 30% by weight of hydrobromic acid were added to a mixture of methyl 2,2-dimethyl-3-(2-cyano-1-propenyl)-cyclopropanecarboxylate (1.0 g) obtained in Production Example 1 and heptane (53.3 g). A part of the obtained mixture was removed and heated with a drier to initiate the isomerization of the compound (1-1) into the compound (2-1). Subsequently, the part was returned again to the mixture, and the mixture was stirred at 25°C for 1 hour.

Analysis of the obtained mixture (reaction mixture) by gas chromatography revealed that the content of the compound (2-1) was 22.7% by weight based on the total amount of the compounds (1-1) and (2-1).

### (Example 9)

Under a nitrogen atmosphere, azobisisobutyronitrile (20.2 mg) and an acetic acid solution (123 mg) containing 30% by weight of hydrobromic acid were added to a mixture of methyl 2,2-dimethyl-3-(2-cyano-1-propenyl)-cyclopropanecarboxylate (1.0 g) obtained in Production Example 1 and heptane (58.2 g). A part of the obtained mixture was removed and heated with a drier to initiate the isomerization of the compound (1-1) into the compound (2-1). Subsequently, the part was returned again to the mixture, and the mixture was stirred at 26°C for 1 hour.

Analysis of the obtained mixture (reaction mixture) by gas chromatography revealed that the content of the compound (2-1) was 13.3% by weight based on the total amount of the compounds (1-1) and (2-1).

### (Example 10)

Under a nitrogen atmosphere, 2,2'-azebis(4-methoxy-2,4-dimethylvaleronitrile) (73.4 mg) and an acetic acid solution (209 mg) containing 30% by weight of hydrobromic acid were added to a mixture of methyl 2,2-dimethyl-3-(2-cyano-1-propenyl)-cyclopropanecarboxylate (1.0 g) obtained in Production Example 1 and heptane (48.8 g). A part of the obtained mixture was removed and heated with a drier to initiate the isomerization of the compound (1-1) into the compound (2-1). Subsequently, the part was returned again to the mixture, and the mixture was stirred at 26°C for 1 hour.

Analysis of the obtained mixture (reaction mixture) by gas chromatography revealed that the content of the compound (2-1) was 49.9% by weight, based on the total amount of the compounds (1-1) and (2-1).

As shown in Examples 1 to 10, the content of the compound (2) based on the total amount of the compounds (1) and (2) increased from 1.9% by weight before the isomerization step to 10.6 to 58.1% by weight by passing through the isomerization step. That is, the compound (2) could be produced from the compound (1) by the isomerization step.

### (Example 11)

Under a nitrogen atmosphere, calcium chloride (2 g) was added to the toluene solution (119 g) obtained in Production Example 2, and a toluene solution (0.83 g) containing t-butyl hydroperoxide in a proportion of 50% by weight and a 47% by weight aqueous solution (2.85 g) of hydrobromic acid were then added dropwise, simultaneously, to the mixture at 40°C over 1 hour. After stirring at the same temperature for 3 hours, the reaction solution was cooled to 25°C and washed by adding distilled water, and then the mixture was separated. To the obtained oil layer, distilled water (42 g) was added and then an aqueous 23% by weight solution (38.2 g) of sodium hydroxide was added dropwise. The mixture was stirred for 30 minutes at an internal temperature of 45°C and then separated into an oil layer I and an aqueous layer I. The oil layer I was further extracted with distilled water to obtain an oil layer II and an aqueous layer II. The aqueous layer I was combined with the aqueous layer II to obtain an aqueous solution containing a sodium salt of 2,2-dimethyl-3-(2-cyano-1-propenyl)-cyclopropanecarboxylic acid. To the aqueous solution of a sodium salt, toluene (54 g) was added and then concentrated sulfuric acid was added dropwise. The mixture was separated into an oil layer III and an aqueous layer III, and the aqueous layer III was extracted by addition of toluene to obtain a toluene layer. The oil layer III was combined with the toluene layer to obtain an oil layer IV. The oil layer IV was washed with distilled water and then dehydrated under reflux to obtain a toluene solution (135 g) of 2,2-dimethyl-3-(2-cyano-1-propenyl)-cyclopropanecarboxylic acid.
Total concentration of compounds (1-161) and (2-161): 25.33%
Total recovery rate of compounds (1-161) and (2-161): 95.4%
Compound (1-161): compound (2-161) = 39.2 : 60.8 (ratio by weight)
Isomer ratio based on 100% by weight in total of compounds (1-161) and (2-161) :
   (1R, 3R)-isomer: 99.9%
   (1R, 3S)-isomer: 0.1%
   (1S, 3R)-isomer: 0.0%
   (1S, 3S)-isomer: 0.0%

### (Example 12)

Under a nitrogen atmosphere, 2,2-dimethyl-3-(2-cyano-1-propenyl)-cyclopropanecarboxylic acid (91.8 g) obtained in Production Example 4 was dissolved in toluene (208.1 g). To the toluene solution, a toluene solution (1.97 g) containing t-butyl hydroperoxide in a proportion of 50% by weight was added in a quarter amount every 15 minutes, and an acetic acid solution (17.0 g) containing hydrobromic acid in a proportion of 20% by weight was added dropwise at 40°C over 1 hour. The mixture was stirred at the same temperature for 2 hours and then washed by addition of water, and the mixture was separated. To the obtained oil layer, water (103.8 g) was added, and a 23% by weight aqueous solution (150.7 g) of sodium hydroxide was added dropwise over 2 hours. Subsequently, the mixture was stirred at the same temperature for 30 minutes and then separated to obtain an aqueous solution containing a sodium salt of 2,2-dimethyl-3-(2-cyano-1-propenyl)-cyclopropanecarboxylic acid. To the aqueous solution of a sodium salt, toluene (134.5 g) was added and concentrated sulfuric acid (35.0 g) was then added dropwise over 3 hours. The mixture was stirred at the same temperature for 30 minutes and then separated to obtain a toluene solution of 2,2-dimethyl-3-(2-cyano-1-propenyl)-cyclopropanecarboxylic acid. After washing with water (35.0 g), the obtained oil layer was subjected to pressure reduction under a pressure of 30 kPa and dehydrated with heating under reflux to obtain a toluene solution (358.19 g) of 2,2-dimethyl-3-(2-cyano-1-propenyl)-cyclopropanecarboxylic acid.
Total concentration of compounds (1-161) and (2-161): 24.7%
Total recovery rate of compounds (1-161) and (2-161): 98.9%
Compound (1-161): compound (2-161) = 40.5 : 59.5 (ratio by weight)
Isomer ratio based on 100% by weight in total of compounds (1-161) and (2-161):
   (1R, 3R)-isomer: 94.9%
   (1R, 3S)-isomer: 2.0%
   (1S, 3R)-isomer: 0.0%
   (1S, 3S)-isomer: 3.1%

As shown in Examples 11 and 12, the content of the compound (2) based on the total amount of the compounds (1) and (2) increased from 1.1% by weight before the isomerization step to 60.8% by weight and 59.5% by weight, respectively, by passing through the isomerization step. That is, the compound (2) could be produced from the compound (1) by the isomerization step. Also, there was almost no change in the steric configuration of position 1 and position 3 in the cyclopropane ring between before and after the isomerization step.

### (Example 13)

To a toluene solution (30 mL) containing 60 mg in total of (Z)-2,2-dimethyl-3-(2-chloro-2-cyano-1-vinyl)-cyclopropanecarboxylic acid (hereinafter sometimes referred to as compound (1-173)) and (E)-2,2-dimethyl-3-(2-chloro-2-cyano-1-vinyl)-cyclopropanecarboxylic acid (hereinafter sometimes referred to as compound (2-173)) in which the ratio by weight of these compounds is compound (1-173) : compound (2-173) = 77 : 33, diphenyl disulfide (12 mg) and benzoyl peroxide (6 mg) were added, and the mixture was refluxed with heating for 2 hours. To the obtained reaction mixture, 1 N hydrochloric acid was added, and the mixture was extracted with ethyl acetate to obtain an ethyl acetate solution containing 55 mg in total of the compound (1-173) and the compound (2-173) (compound (1-173) : compound (2-173) = 40 : 60 (ratio by weight)). The recovery rate of the compound (1-173) and the compound (2-173) was 92%.

As shown in Example 13, the content of the compound (2) based on the total amount of the compounds (1) and (2) increased from 33% by weight before the isomerization step to 60% by weight by passing through the isomerization step. That is, the compound (2) could be produced from the compound (1) by the isomerization step.

### (Example 14)

The compound (2) can be produced from the compound
(1) even when using, instead of bromine used in Example 1, the following isomerizing catalysts including:
   acetyl bromide, propionyl bromide, benzoyl bromide, phosphorus pentaoxide, N-bromosuccinimide, N-bromoacetamide, trimethylsilyl bromide, silicon tetrabromide, thionyl bromide, boron tribromide, aluminum tribromide, thiophenol, p-thiocresol, 1-butanethiol, thiobenzoic acid, thioacetic acid, thiosalicylic acid, nitric acid, and sodium nitrate.

### INDUSTRIAL APPLICABILITY

It is known that the compound (2) is useful as an agent for controlling noxious organisms such as pests. The present invention can be industrially used as a method for producing the compound (2).

## Claims

1. A method for producing a (Z)-cyanoalkenyl-cyclopropanecarboxylic acid compound, which method comprises the step of isomerizing an (E)-cyanoalkenyl-cyclopropanecarboxylic acid compound represented by formula (1) : 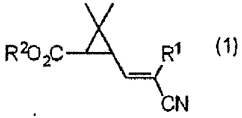 wherein R¹ represents an alkyl group which may have a substituent, or a halogen atom, and R² represents a hydrogen atom, an alkyl group which may have a substituent, or a benzyl group which may have a substituent,
into a (Z)-cyanoalkenyl-cyclopropanecarboxylic acid compound represented by formula (2): 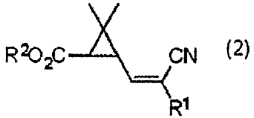 wherein R¹ and R² have the same meanings as defined above, in the presence of at least one isomerizing catalyst selected from the group consisting of bromine, hydrogen bromide, brominated carboxylic acids, brominated phosphorus compounds, N-brominated imide compounds, N-brominated amide compounds, brominated alkylsilane compounds, thionyl bromide, brominated boron compounds, brominated aluminum compounds, thiol compounds, disulfide compounds, thiocarboxylic acid compounds, nitric acid and nitrate salts.

2. The method according to claim 1, wherein the above described step is the step of isomerizing the (E)-cyanoalkenyl-cyclopropanecarboxylic acid compound into the (Z)-cyanoalkenyl-cyclopropanecarboxylic acid compound further in the presence of a radical initiator.

3. The method according to claim 2, wherein the radical initiator is at least one radical initiator selected from the group consisting of inorganic peroxides, ketone peroxide compounds, hydroperoxide compounds, diacyl peroxide compounds, peracid ester compounds, peracid compounds and azo compounds.

4. The method according to claim 1 or 2, wherein R² represents a hydrogen atom.

5. The method according to claim 1 or 2, wherein R² represents an alkyl group which may have a substituent, or a benzyl group which may have a substituent.

6. A method for isomerizing an (E)-cyanoalkenyl-cyclopropanecarboxylic acid compound, which comprises isomerizing an (E)-cyanoalkenyl-cyclopropanecarboxylic acid compound represented by formula (1): 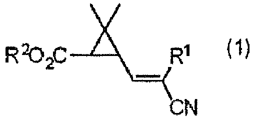 wherein R¹ represents an alkyl group which may have a substituent, or a halogen atom, and R² represents a hydrogen atom, an alkyl group which may have a substituent, or a benzyl group which may have a substituent,
into a (Z)-cyanoalkenyl-cyclopropanecarboxylic acid compound represented by formula (2): 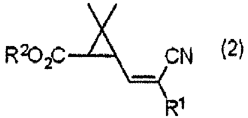 wherein R¹ and R² have the same meanings as defined above, in the presence of at least one isomerizing catalyst selected from the group consisting of bromine, hydrogen bromide, brominated carboxylic acids, brominated phosphorus compounds, N-brominated imide compounds, N-brominated amide compounds, brominated alkylsilane compounds, thionyl bromide, brominated boron compounds, brominated aluminum compounds, thiol compounds, disulfide compounds, thiocarboxylic acid compounds, nitric acid and nitrate salts.

## Patentansprüche

1. Ein Verfahren zur Herstellung einer (Z)-Cyanoalkenyl-Cyclopropancarbonsäureverbindung, wobei das Verfahren den Schritt der Isomerisierung einer (E)-Cyanoalkenyl-Cyclopropancarbonsäureverbindung, dargestellt durch die Formel (1): 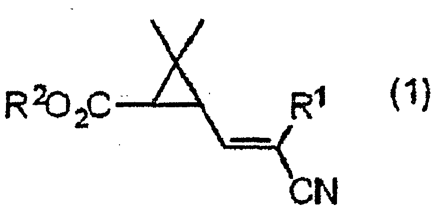 wobei R¹ eine Alkylgruppe, welche einen Substituenten aufweisen kann, oder ein Halogenatom darstellt, und R² ein Wasserstoffatom, eine Alkylgruppe, welche einen Substituenten aufweisen kann, oder eine Benzylgruppe, welche einen Substituenten aufweisen kann, darstellt,
in eine (Z)-Cyanoalkenyl-Cyclopropancarbonsäureverbindung, dargestellt durch die Formel (2): 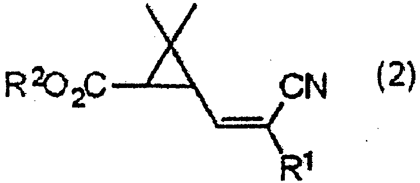 wobei R¹ und R² die vorstehend definierten Bedeutungen haben,
in Gegenwart von mindestens einem Isomerisierungskatalysator ausgewählt aus der Gruppe bestehend aus Brom, Bromwasserstoff, bromierten Carbonsäuren, bromierten Phosphorverbindungen, N-bromierten Imidverbindungen, N-bromierten Amidverbindungen, bromierten Alkylsilanverbindungen, Thionylbromid, bromierten Borverbindungen, bromierten Aluminiumverbindungen, Thiolverbindungen, Disulfidverbindungen, Thiocarbonsäureverbindungen, Salpetersäure und Nitratsalzen, umfasst.

2. Das Verfahren gemäß Anspruch 1, wobei der vorstehend beschriebene Schritt der Schritt der Isomerisierung der (E)-Cyanoalkenyl-Cyclopropancarbonsäureverbindung in die (Z)-Cyanoalkenyl-Cyclopropancarbonsäureverbindung, ferner in Gegenwart eines Radikalinitiators ist.

3. Das Verfahren gemäß Anspruch 2, wobei der Radikalinitiator mindestens ein Radikalinitiator ausgewählt aus der Gruppe bestehend aus anorganischen Peroxiden, Ketonperoxidverbindungen, Hydroperoxidverbindungen, Diacylperoxidverbindungen, Persäureesterverbindungen, Persäureverbindungen und Azoverbindungen ist.

4. Das Verfahren gemäß Anspruch 1 oder 2, wobei R² ein Wasserstoffatom darstellt.

5. Das Verfahren gemäß Anspruch 1 oder 2, wobei R² eine Alkylgruppe, welche einen Substituenten aufweisen kann, oder eine Benzylgruppe, welche einen Substituenten aufweisen kann, darstellt.

6. Ein Verfahren zur Isomerisierung einer (E)-Cyanoalkenyl-Cyclopropancarbonsäureverbindung, welches die Isomerisierung einer (E)-Cyanoalkenyl-Cyclopropancarbonsäureverbindung, dargestellt durch die Formel (1): 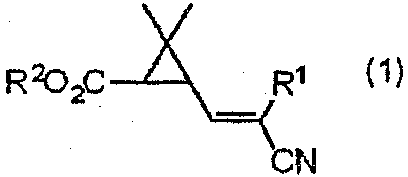 wobei R¹ eine Alkylgruppe, welche einen Substituenten aufweisen kann, oder ein Halogenatom darstellt, und R² ein Wasserstoffatom, eine Alkylgruppe, welche einen Substituenten aufweisen kann, oder eine Benzylgruppe, welche einen Substituenten aufweisen kann, darstellt, in eine (Z)-Cyanoalkenyl-Cyclopropancarbonsäureverbindung, dargestellt durch die Formel (2): 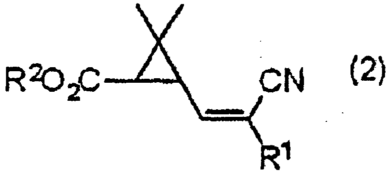 wobei R¹ und R² die vorstehend definierten Bedeutungen haben,
in Gegenwart von mindestens einem Isomerisierungskatalysator ausgewählt aus der Gruppe bestehend aus Brom, Bromwasserstoff, bromierten Carbonsäuren, bromierten Phosphorverbindungen, N-bromierten Imidverbindungen, N-bromierten Amidverbindungen, bromierten Alkylsilanverbindungen, Thionylbromid, bromierten Borverbindungen, bromierten Aluminiumverbindungen, Thiolverbindungen, Disulfidverbindungen, Thiocarbonsäureverbindungen, Salpetersäure und Nitratsalzen, umfasst.

## Revendications

1. Procédé de production d'un composé d'acide (Z)-cyanoalcénylcyclopropanecarboxylique, le procédé comprenant l'étape d'isomérisation d'un composé d'acide (E)-cyanoalcényl-cyclopropanecarboxylique représenté par la formule (1) : 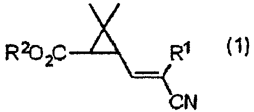 dans laquelle R¹ représente un groupe alkyle qui peut porter un substituant, ou un atome d'halogène, et R² représente un atome d'hydrogène, un groupe alkyle qui peut porter un substituant, ou un groupe benzyle qui peut porter un substituant,
en composé d'acide (Z)-cyanoalcénylcyclopropane-carboxylique représenté par la formule (2) : 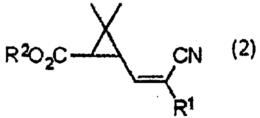 dans laquelle R¹ et R² ont les mêmes significations que celles définies ci-dessus, en présence d'au moins un catalyseur d'isomérisation choisi dans le groupe constitué par un atome de brome, un bromure d'hydrogène, des acides carboxyliques bromés, des composés de phosphore bromés, des composés d'imide N-bromés, des composés d'amide N-bromés, des composés d'alkylsilane bromés, un bromure de thionyle, des composés de bore bromés, des composés d'aluminium bromés, des composés de thiol, des composés de disulfure, des composés d'acide thiocarboxylique, l'acide nitrique et des sels de nitrate.

2. Procédé selon la revendication 1, dans lequel l'étape décrite ci-dessus est l'étape d'isomérisation du composé d'acide (E)-cyanoalcénylcyclopropanecarboxylique en composé d'acide (Z)-cyanoalcénylcyclopropanecarboxylique en présence en outre d'un amorceur radicalaire.

3. Procédé selon la revendication 2, dans lequel l'amorceur radicalaire est au moins un amorceur radicalaire choisi dans le groupe constitué par les peroxydes inorganiques, les composés de peroxyde de cétone, les composés d'hydroperoxyde, les composés de peroxyde de diacyle, les composés d'ester peracide, les composés peracides et les composés azoïques.

4. Procédé selon la revendication 1 ou 2, dans lequel R² représente un atome d'hydrogène.

5. Procédé selon la revendication 1 ou 2, dans lequel R² représente un groupe alkyle qui peut porter un substituant, ou un groupe benzyle qui peut porter un substituant.

6. Procédé d'isomérisation d'un composé d'acide (E)-cyanoalcénylcyclopropanecarboxylique, qui comprend l'isomérisation d'un composé d'acide (E)-cyanoalcényl-cyclopropanecarboxylique représenté par la formule (1) : 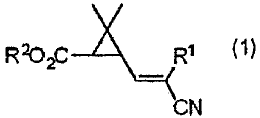 dans laquelle R¹ représente un groupe alkyle qui peut porter un substituant, ou un atome d'halogène, et R² représente un atome d'hydrogène, un groupe alkyle qui peut porter un substituant, ou un groupe benzyle qui peut porter un substituant,
en composé d'acide (Z)-cyanoalcénylcyclopropanecarboxylique représenté par la formule (2) : 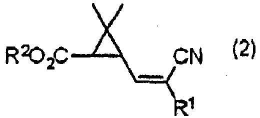 dans laquelle R¹ et R² ont les mêmes significations que celles définies ci-dessus, en présence d'au moins un catalyseur d'isomérisation choisi dans le groupe constitué par un atome de brome, un bromure d'hydrogène, des acides carboxyliques bromés, des composés de phosphore bromés, des composés d'imide N-bromés, des composés d'amide N-bromés, des composés d'alkylsilane bromés, un bromure de thionyle, des composés de bore bromés, des composés d'aluminium bromés, des composés de thiol, des composés de disulfure, des composés d'acide thiocarboxylique, l'acide nitrique et des sels de nitrate.
